(19) Europäisches Patentamt
European Patent Office
Office européen des brevets

(11) Veröffentlichungsnummer: **0 117 482**
**B1**

(12) # EUROPÄISCHE PATENTSCHRIFT

(45) Veröffentlichungstag der Patentschrift: **17.09.86**

(51) Int. Cl.⁴: **C 07 D 207/456,** A 01 N 43/36

(21) Anmeldenummer: **84101526.6**

(22) Anmeldetag: **15.02.84**

(54) **Substituierte Maleinsäureimide und deren Verwendung als Schädlingsbekämpfungsmittel.**

(30) Priorität: **25.02.83 DE 3306697**

(43) Veröffentlichungstag der Anmeldung:
**05.09.84 Patentblatt 84/36**

(45) Bekanntmachung des Hinweises auf die Patenterteilung:
**17.09.86 Patentblatt 86/38**

(84) Benannte Vertragsstaaten:
**AT BE CH DE FR GB IT LI NL SE**

(56) Entgegenhaltungen:
**EP - A - 0 045 907**
**EP - A - 0 098 953**
**GB - A - 880 555**
**GB - A - 2 087 879**
**US - A - 3 129 225**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Marzolph, Gerhard, Dr., Semmelweisstrasse 87a, D-5000 Köln 80 (DE)**
Erfinder: **Blank, Heinz Ulrich, Dr., Am Geusfelde 35, D-5068 Odenthal (DE)**
Erfinder: **Reinecke, Paul, Dr., Lessingstrasse 11, D-5090 Leverkusen 3 (DE)**
Erfinder: **Brandes, Wilhelm, Dr., Eichendorffstrasse 3, D-5653 Leichlingen 1 (DE)**
Erfinder: **Hänssler, Gerd, Dr., Heymannstrasse 40, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 117 482 B1**

ACTORUM AG

**Beschreibung**

Die vorliegende Erfindung betrifft neue substituierte Maleinsäureimide, mehrere Verfahren zu ihrer Herstellung und ihre Verwendung als Schädlingsbekämpfungsmittel.

Es ist bereits bekannt, dass N-Aryl-maleinsäureimide, wie z.B. das 2,3-Dichlormaleinsäure-N-(4--fluorphenyl)-imid, das 2,3-Dichlormaleinsäure-N--(4-difluormethoxyphenyl)-imid oder das 2,3-Dichlormaleinsäure-N-phenyl-imid, fungizide Eigenschaften besitzen (vgl. US-Patent 3734927, GB-A 2087879 und GB-Patent 880555).

Weiterhin sind N-Aralkyl-maleinsäureimide, wie z.B. das Maleinsäure-N-(2-phenyl-ethyl)-imid, bekannt, die als Saatgutbeizmittel eingesetzt werden (vgl. Jap. Patentanmeldung 78-27336) und N--Alkyl- bzw. N-Cycloalkyl-maleinsäureimide, die als Mikrobizide bekannt sind (vgl. EP-A 0045907).

Es wurden neue substituierte Maleinsäureimide der Formel (I) gefunden

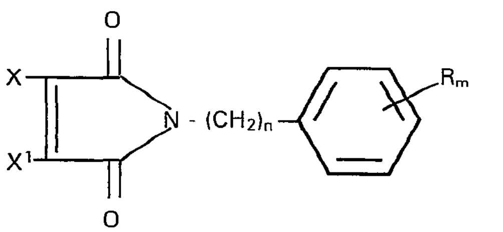
(I)

in welcher

X für Wasserstoff, Chlor oder Brom,

$X^1$ für Chlor oder Brom,

n für 2, 3 oder 4,

R für Halogen, Alkyl mit 1 bis 6 Kohlenstoffatomen, gegebenenfalls ein- bis fünffach, gleich oder verschieden durch Halogen und Alkyl substituiertes Aryl, Cycloalkyl oder Aryloxy, für Alkoxy, Alkoxycarbonyl, Alkylcarbonyl oder Alkylsulfonyl mit 1 bis 5 Kohlenstoffatomen je Alkylrest, für Carboxy, Aminocarbonyl, Aminosulfonyl, Nitro, Cyan, Hydroxy, Halogenalkyl mit 1 bis 7 Halogenatomen und 1 bis 4 Kohlenstoffatomen je Alkylrest, für Acyloxy oder Acylamino mit 1 bis 5 Kohlenstoffatomen je Acylrest, Amino, Mono- und Dialkylamino mit 1 bis 4 Kohlenstoffatomen je Alkylrest und

m für 1, 2, 3, 4 oder 5 steht.

Weiterhin wurde gefunden, dass man die substituierten Maleinsäureimide der Formel (I)

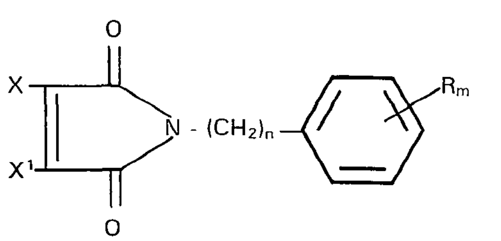
(I)

in welcher

X für Wasserstoff, Chlor oder Brom,

$X^1$ für Chlor oder Brom,

n für 2, 3 oder 4,

R für Halogen, Alkyl mit 1 bis 6 Kohlenstoffatomen, gegebenenfalls ein- bis fünffach, gleich oder verschieden durch Halogen und Alkyl substituiertes Aryl, Cycloalkyl oder Aryloxy, für Alkoxy, Alkoxycarbonyl, Alkylcarbonyl oder Alkylsulfonyl mit 1 bis 5 Kohlenstoffatomen je Alkylrest, für Carboxy, Aminocarbonyl, Aminosulfonyl, Nitro, Cyan, Hydroxy, Halogenalkyl mit 1 bis 7 Halogenatomen und 1 bis 4 Kohlenstoffatomen je Alkylrest, für Acyloxy oder Acylamino mit 1 bis 5 Kohlenstoffatomen je Acylrest, Amino, Mono- und Dialkylamino mit 1 bis 4 Kohlenstoffatomen je Alkylrest und

m für 1, 2, 3, 4 oder 5 steht

erhält, wenn man

a) ein Halogenmaleinsäureanhydrid der Formel (II)

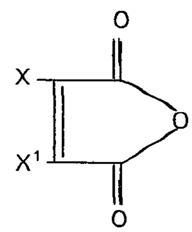
(II)

in welcher

X und $X^1$ die oben angegebene Bedeutung haben, mit primären Aminen der Formel (III)

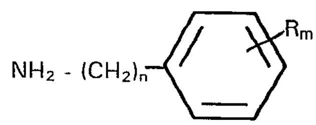
(III)

in welcher

R, m und n die oben angegebene Bedeutung haben, in einem Verdünnungsmittel umsetzt, oder

b) einen Halogenmaleinsäuredialkylester der Formel (IV)

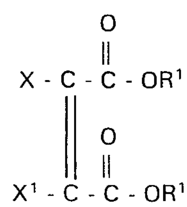
(IV)

in welcher

X und $X^1$ die oben angegebene Bedeutung haben, und

$R^1$ für Alkyl steht,

mit primären Aminen der Formel (III), in welcher

R, m und n die oben angegebene Bedeutung haben, gegebenenfalls in einem Lösungs- oder Verdünnungsmittel umsetzt, oder

c) Halogenmaleinsäuremonoamide der Formel (V)

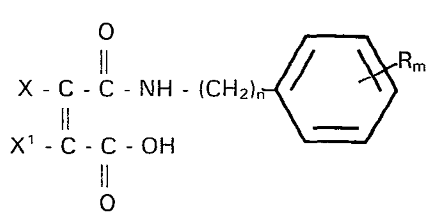
(V)

in welcher

X, $X^1$, R, m und n die oben angegebene Bedeutung haben,
in Gegenwart eines Lösungsmittels, wie z.B. Eisessig, und gegebenenfalls eines anhydridisierenden Mittels, wie z.B. Acetanhydrid oder Thionylchlorid, zu den Verbindungen der Formel (I) cyclisiert.

Die neuen substituierten Maleinsäureimide weisen starke fungizide Eigenschaften auf. Überraschenderweise zeigen dabei die erfindungsgemässen Verbindungen der Formel (I) eine erheblich höhere Wirkung als die aus dem Stand der Technik bekannten Verbindungen, welche wirkungsmässig naheliegende Verbindungen sind.

Besonders bevorzugt sind solche substituierten Maleinsäureimide der Formel (I), in welcher
X für Wasserstoff, Chlor oder Brom,
$X^1$ für Chlor oder Brom,
n für 2, 3 oder 4,
R für Fluor, Chlor, Brom, Alkyl mit 1 bis 5 Kohlenstoffatomen, für Alkoxy mit 1 bis 4 Kohlenstoffatomen, für Aminosulfonyl, Alkylsulfonyl bzw. Alkylcarbonyl mit 1 bis 3 Kohlenstoffatomen im Alkylteil, für Aminocarbonyl, für Halogenalkyl mit 1 bis 3 Kohlenstoffatomen und 1 bis 5 Halogenatomen, Phenyl, Cyclopentyl oder Cyclohexyl, für Alkoxycarbonyl mit 1 bis 4 Kohlenstoffatomen im Alkoxyteil, für Hydroxy, für Acyloxy bzw. Acylamino mit 1 bis 3 Kohlenstoffatomen je Acylrest, für Amino, Mono- bzw. Dialkylamino mit 1 bis 3 Kohlenstoffatomen je Alkylrest, Nitro, Chlorphenyl und
m für 1, 2, 3, 4 oder 5 stehen.

Ganz besonders bevorzugt sind solche substituierten Maleinsäureimide der Formel (I), in welcher
X und $X^1$ für Chlor oder Brom,
n für 2 oder 3,
R für Fluor, Chlor, Brom, Methyl, Ethyl, n-Propyl, iso-Propyl, n-Butyl, sec.-Butyl, iso-Butyl und tert.-Butyl, für Methoxy, Ethoxy, n-Propoxy und iso-Propoxy, für Aminosulfonyl, für Methylsulfonyl, Ethylsulfonyl, n-Propylsulfonyl, iso-Propylsulfonyl, Methylcarbonyl, Ethylcarbonyl, n-Propylcarbonyl und iso-Propylcarbonyl, für Aminocarbonyl, Trifluormethyl, Phenyl, Cyclopentyl oder Cyclohexyl, für Methoxycarbonyl, Ethoxycarbonyl, n-Propoxycarbonyl und iso-Propoxycarbonyl, für Hydroxy, für Formyloxy, Acetoxy, Formylamino und Acetamido, für Amino, für Methylamino, Dimethylamino, Ethylamino, Diethylamino, Nitro und
m für 1, 2 oder 3 stehen.

Verwendet man beispielsweise für die Verfahrensvariante a) Dibrommaleinsäureanhydrid und 3-(4-Chlorphenyl)-propylamin, so kann der Reaktionsverlauf durch das folgende Formelschema wiedergegeben werden:

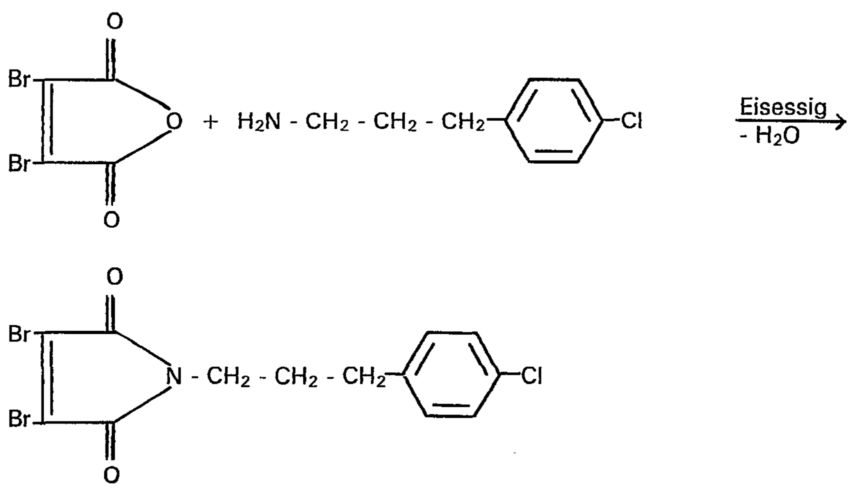


Verwendet man bei der Verfahrensvariante b) Dichlormaleinsäuredimethylester und 2-(3-Trifluormethylphenyl)-ethylamin als Ausgangsstoffe, so kann der Reaktionsverlauf durch das folgende Formelschema wiedergegeben werden:

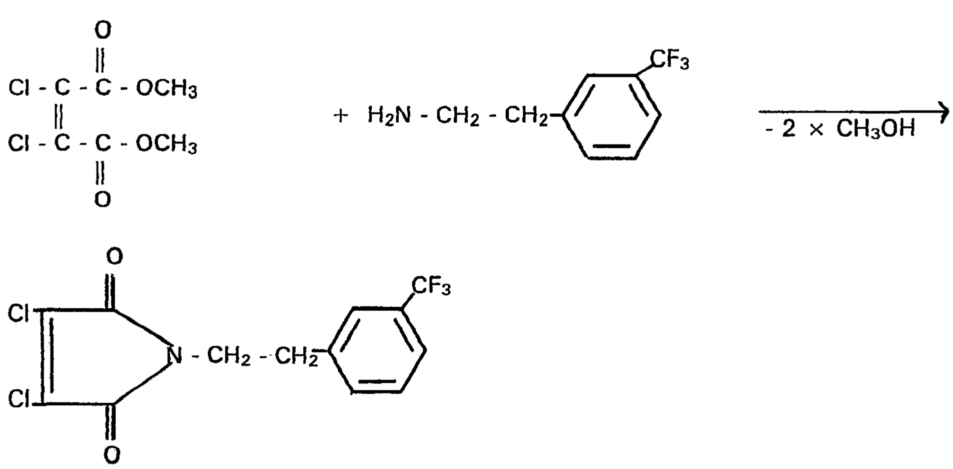

Verwendet man bei der Verfahrensvariante c) Dichlormaleinsäureanhydrid und 2-(2-Chlor-phenyl)-ethylamin als Ausgangsstoffe, so erhält man die Dichlormaleinsäuremonoamide, die cyclisiert werden. Dieser Reaktionsverlauf kann durch das folgende Formelschema wiedergegeben werden:

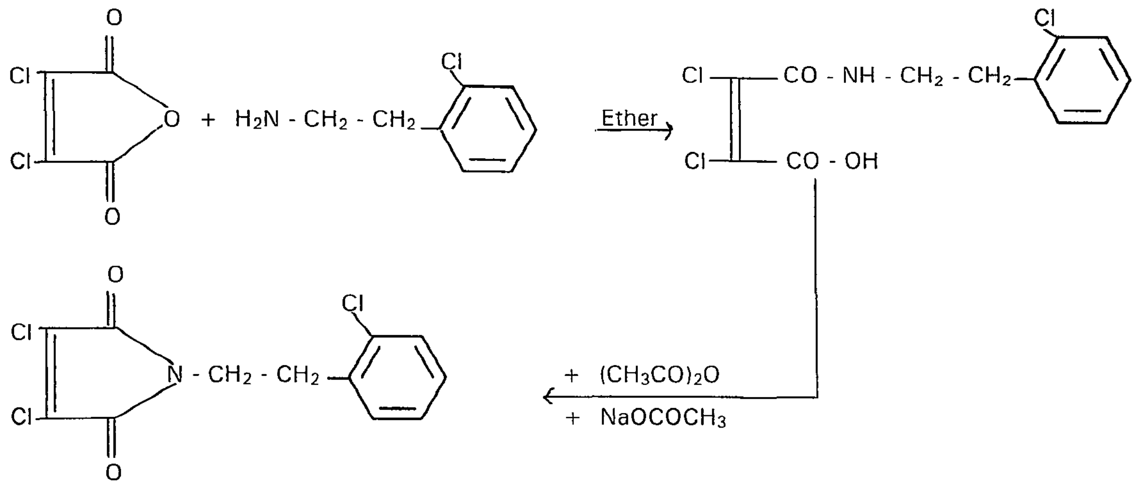


Die bei der Durchführung der Verfahrensvariante a) als Ausgangsverbindungen benötigten Halogenmaleinsäureanhydride sind durch die Formel (II) allgemein definiert. Diese Verbindungen sind als Säuren oder Anhydride im Handel erhältlich und/oder nach bekannten Verfahren herstellbar. Die weiterhin bei den Verfahrensvarianten a) und b) einzusetzenden Amine sind durch die Formel (III) definiert. In dieser Formel haben die Reste, die bereits im Zusammenhang mit der Beschreibung der erfindungsgemässen Stoffe der Formel (I) gegebene Bedeutung. Die Amine sind teilweise bekannt oder können nach allgemein bekannten Verfahren hergestellt werden. So können die Amine beispielsweise durch Reduktion von Nitrolen oder Aldoximen mit Wasserstoff hergestellt werden (vgl. Houben-Weyl, Methoden der organischen Chemie, XI/1, Seiten 341 ff). Weiterhin ist eine gängige Methode die reduktive Aminierung von Aldehyden mit Wasserstoff und Ammoniak (vgl. Houben-Weyl, XI/1, Seite 341). Zur Herstellung von Phenylethylaminen eignet sich vor allem die Methode der Reduktion von ω-Nitrostyrolen mit komplexen Hydriden (vgl. Canad. J. of Chem. 51, 1973, Seite 1402).

Die bei der Verfahrensvariante b) noch benötigten Halogenmaleinsäuredialkylester sind durch die Formel (IV) beschrieben. Diese Ester sind bekannt und nach gängigen Verfahren aus den käuflichen Halogenmaleinsäureanhydriden durch Umsetzung mit Alkoholen erhältlich.

Die bei der Verfahrensvariante c) einzusetzenden Halogenmaleinsäuremonoamide der Formel (V) sind teilweise bekannt oder können nach an sich bekannten Verfahren aus den entsprechenden Maleinsäureanhydriden durch Umsetzung mit primären Aminen hergestellt werden (vgl. Organic Synthesis 41, Seite 93 [1961]).

Als Verdünnungsmittel kommen für die Verfahrensvariante a) vor allem Carbonsäuren in Frage, wie z.B. Ameisensäure, Essigsäure und Propionsäure.

Als Verdünnungsmittel kommen bei der Verfahrensvariante b) organische Lösungsmittel in Frage. Hierzu gehören vorzugsweise Toluol, Xylol, Chlorbenzol, Perchlorethan, Dioxan, Glykoldimethylether, Dimethylformamid.

Bei der Verfahrensvariante c) werden vorzugsweise als Verdünnungsmittel eingesetzt: Carbonsäure, wie Essigsäure; aromatische Kohlenwasserstoffe, wie Toluol, Xylol; Halogenkohlenwasserstoffe, wie Chlorbenzol; weiterhin Dioxan und als anhydridisierende Reagenzien vorzugsweise Acetanhydrid, Phosgen, Thionylchlorid, Phosphoroxichlorid, Phosphorpentachlorid.

Die Reaktionstemperaturen können bei der Durchführung der verschiedenen Verfahrensvarianten in einem grösseren Bereich variiert werden. Bei Verfahrensvariante a) arbeitet man bei Temperaturen von 20 bis 150°C, vorzugsweise von 80 bis 120°C. Bei Verfahrensvariante b) arbeitet man bei Temperaturen von 50 bis 180°C, vorzugsweise von 80 bis 130°C und bei der Verfahrensvariante c) bei Temperaturen von 0 bis 150°C, vorzugsweise von 50 bis 120°C.

Bei allen drei Varianten wird im allgemeinen bei Normaldruck gearbeitet.

Bei der Durchführung aller Verfahrensvarianten werden die Ausgangsstoffe vorzugsweise in äquimolaren Mengen eingesetzt.

Die Amine der Formel (III) können als freie Basen oder in Form ihrer Salze, wie z.B. der Hydrochloride, Hydrobromide, Acetate oder Oxalate, eingesetzt werden.

Bei Verwendung der Amine der Formel (III) in Form ihrer Salze wird zweckmässigerweise eine entsprechende z.B. äquimolare Menge einer Hilfsbase, wie z.B. Triethylamin oder Natriumacetat, dem Reaktionsgemisch zugesetzt.

Nach einer bevorzugten Ausführungsform der Verfahrensvariante a) werden die Ausgangsstoffe in äquimolaren Mengen in einem organischen Lösungsmittel, z.B. Eisessig, mehrere Stunden bei erhöhter Temperatur gerührt. Anschliessend auf Raumtemperatur abgekühlt, Wasser wird zugesetzt, wobei das Produkt bereits ausgeschieden wird.

Setzt man das Dibrommaleinsäureanhydrid als Ausgangsstoff ein, so wird dieses in einer bevorzugten Ausführungsform in der Lösung aus Dibrommaleinsäure in Eisessig unter Rühren hergestellt und in dieser Lösung direkt weiter mit dem Amin umgesetzt.

Nach einer bevorzugten Ausführungsform der Verfahrensvariante b) wird der Dihalogenmaleinsäuredialkylester aus Dihalogenmaleinsäureanhydrid und Methanol hergestellt und dieser nach fraktionierter Destillation mit dem Amin umgesetzt. Die Aufarbeitung erfolgt wie oben beschrieben (vgl. US-Patent 3 734 927, Beispiel 5).

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemässen Wirkstoffe mit besonders gutem Erfolg zur Bekämpfung von Venturia-Arten, z.B. gegen den Erreger des Apfelschorfs (Venturia inaequalis), zur Bekämpfung von Leptosphaeria-Arten, wie z.B. den Erreger der Braunfleckenkrankheit des Weizens (Leptosphaeria nodorum), zur Bekämpfung von Oomyceten, wie z.B. den Erreger der Kartoffel- und Tomatenkrautfäule (Phytophthora infestans), zur Bekämpfung von Reiskrankheiten, wie z.B. Pellicularia sasakii und Pyricularia oryzae, zur Bekämpfung von Puccinia-Arten, z.B. gegen den Erreger des Weizenbraunrostes (Puccinia recondita), eingesetzt werden.

Weiterhin ist die fungizide Wirkung gegen Cochliobolus sativus und Pyrenophora teres an Getreide zu nennen. Bei entsprechenden Konzentrationen sind die Verbindungen auch akarizid wirksam.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-ether, z.B. Alkylarylpolyglykolether, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90%.

Die erfindungsgemässen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Giessen, Tauchen, Spritzen, Sprühen, Vernebeln, Verdampfen, Injizieren, Verschlämmen, Verstreichen, Stäuben, Streuen, Trockenbeizen,

Feuchtbeizen, Nassbeizen, Schlämmbeizen oder Inkrustieren.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei der Behandlung des Bodens sind Wirkstoffkonzentrationen von 0,00001 bis 0,1 Gew.-%, vorzugsweise von 0,0001 bis 0,02%, am Wirkungsort erforderlich.

*Herstellungsbeispiele*

*Beispiel 1*

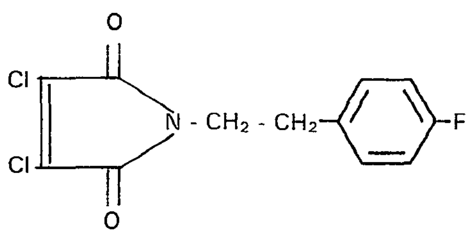

16,7 g (0,1 Mol) Dichlormaleinsäureanhydrid und 14,0 g (0,1 Mol) 2-(4-Fluorphenyl)-ethylamin werden in 100 ml Eisessig 4 Stunden bei 120°C gerührt. Man kühlt auf 20°C ab und gibt 10 ml Wasser zu. Es fällt ein farbloser Niederschlag aus, der abgesaugt und getrocknet wird. Man erhält 16 g Dichlormaleinsäure-N-[2-(4-fluorphenyl)-ethyl]-imid mit dem Schmelzpunkt 137-138°C. Durch Verrühren der Mutterlauge mit Wasser können noch weitere 5,1 g Imid isoliert werden. Die Gesamtausbeute beträgt: 73% der Theorie.

*Beispiel 2*

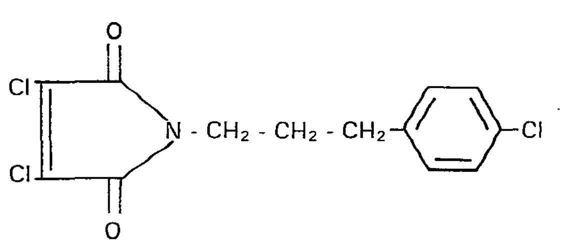

16,7 g (0,1 Mol) Dichlormaleinsäureanhydrid und 17,0 g (0,1 Mol) 3-(4-Chlorphenyl)-propylamin werden in 100 ml Eisessig 4 Stunden am Rückfluss gerührt. Man gibt 50 ml Wasser zu und kühlt auf 20°C ab. Das Produkt wird abgesaugt und getrocknet. Man erhält 26,1 g (82% der Theorie) Dichlormaleinsäure-N-[3-(4-chlorphenyl)-propyl]-imid mit einem Schmelzpunkt von 72-73°C.

*Beispiel 3*

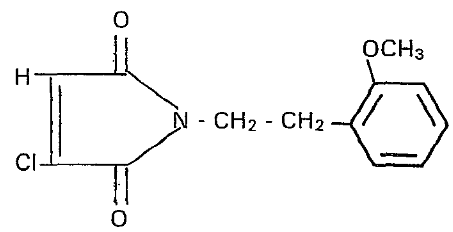

13,25 g (0,1 Mol) Chlormaleinsäureanhydrid und 15,2 g (0,1 Mol) 2-(2-Methoxyphenyl)-ethylamin werden in 100 ml Eisessig 4 Stunden zum Sieden erhitzt. Dann werden 100 ml Wasser zugesetzt und das ausgefallene Öl mit Methylenchlorid extrahiert. Die Methylenchloridphase wird abgetrennt, mit Wasser gewaschen, getrocknet und eingedampft. Das verbleibende Öl kristallisiert. Man erhält 20,8 g (78% der Theorie) Chlormaleinsäure-N-[2-(2-methoxyphenyl)-ethyl]-imid.

*Beispiel 4*

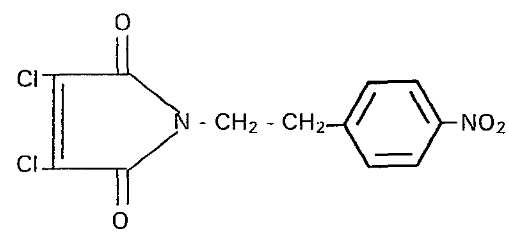

16,7 g (0,1 Mol) Dichlormaleinsäureanhydrid und 16,5 g (0,1 Mol) (4-Nitrophenyl)-ethylamin werden in 100 ml Eisessig 4 Stunden bei Rückfluss gerührt. Man gibt 100 ml Wasser zu, extrahiert das ausgefallene Öl mit Methylenchlorid, engt die Methylenchloridphase ein und kristallisiert den Rückstand aus Ethanol um. Man erhält 25,4 g (81% der Theorie) Dichlormaleinsäure-N-[2-(2-nitrophenyl)-ethyl]-imid mit dem Schmelzpunkt 166°C.

*Beispiel 5*

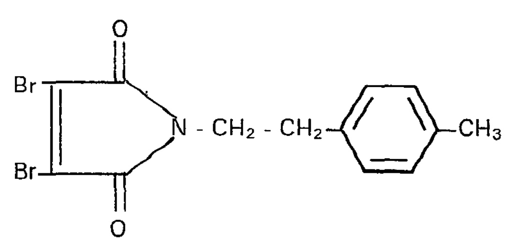

14 g (0,051 Mol) Dibrommaleinsäure werden in 100 ml Eisessig 1 Stunde bei Rückfluss gerührt. In die erkaltete Lösung des so hergestellten Dibrommaleinsäureanhydrids gibt man 6,75 g (0,05 Mol) 2-(p-Tolyl)-ethylamin und rührt weitere 3 Stunden bei Rückfluss. Man kühlt auf 20°C und saugt den

Niederschlag ab. Nach dem Trocknen erhält man 15,5 g (65% der Theorie) Dibrommaleinsäure-N-2-(p-tolyl)-ethylimid.

Analog einem der unter 1-5 beschriebenen Beispiele können die nachfolgend aufgeführten Verbindungen der Formel (I) hergestellt werden:

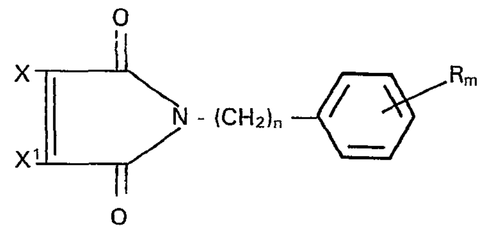

(I)

| Beispiel-Nr. | $X_1$ | $X_2$ | n | R | m | Schmelzpunkt [°C] |
|---|---|---|---|---|---|---|
| 6 | Cl | Cl | 2 | 2-F | 1 | 121 - 122 |
| 7 | Br | Br | 2 | $2,6-Cl_2$ | 2 | 164 - 168 |
| 8 | Cl | Cl | 2 | $2,4-F_2$ | 2 | 132 - 133 |
| 9 | Cl | Cl | 2 | 2-Cl | 1 | 129 - 130 |
| 10 | Cl | Cl | 2 | 3-Cl | 1 | 141 - 142 |
| 11 | Cl | Cl | 2 | 4-Cl | 1 | 118 - 119 |
| 12 | Cl | Cl | 2 | 2-F, 4-Cl | 2 | 122 - 123 |
| 13 | Cl | Cl | 2 | 2-F, 6-Cl | 2 | 121 - 122 |
| 14 | Cl | Cl | 2 | 2-Cl, 4-F | 2 | 124 - 126 |
| 15 | Cl | Cl | 2 | $2,3-Cl_2$ | 2 | 142 - 143 |
| 16 | Cl | Cl | 2 | $2,4-Cl_2$ | 2 | 148 - 149 |
| 17 | Cl | Cl | 2 | $2,5-Cl_2$ | 2 | 133 - 134 |
| 18 | Cl | Cl | 2 | $2,6-Cl_2$ | 2 | 130 - 131 |
| 19 | Cl | Cl | 2 | $3,4-Cl_2$ | 2 | 153 - 154 |
| 20 | Cl | Cl | 2 | $3,5-Cl_2$ | 2 | |
| 21 | Cl | Cl | 2 | 4-Br | 1 | 122 - 123 |
| 22 | Cl | Cl | 2 | $2-OCH_3$ | 1 | 108 |
| 23 | Cl | Cl | 2 | $4-OCH_3$ | 1 | 136 - 138 |
| 24 | Cl | Cl | 2 | 4-OH | 1 | 175 |
| 25 | Cl | Cl | 2 | $4-OCOCH_3$ | 1 | 176 |
| 26 | Cl | Cl | 2 | $3,4-(OCH_3)_2$ | 2 | 160 - 161 |
| 27 | Cl | Cl | 2 | $4-SO_2-NH_2$ | 1 | 213 |
| 28 | Cl | Cl | 2 | $4-NO_2$ | 1 | 166 |
| 29 | Cl | Cl | 2 | $3-CF_3$ | 1 | 87 - 88 |
| 30 | Cl | Cl | 2 | $4-CF_3$ | 1 | 144 - 145 |
| 31 | Cl | Cl | 2 | $4-CH_3$ | 1 | 138 - 139 |
| 32 | Cl | Cl | 2 | $4-C(CH_3)_3$ | 1 | 130 |
| 33 | Cl | Cl | 3 | 4-Cl | 1 | 72 - 73 |
| 34 | Cl | Cl | 3 | $4-OCH_3$ | 1 | Öl |
| 35 | Cl | Cl | 3 | $2,3-Cl_2$ | 2 | 112 - 116 |
| 36 | Cl | Cl | 3 | $3,4-Cl_2$ | 2 | 92 - 95 |
| 37 | Cl | Cl | 3 | 2-Cl | 1 | |
| 38 | Cl | Cl | 3 | $3-CF_3$ | 1 | |
| 39 | Cl | Cl | 3 | 2-F, 4-Cl | 2 | 67 - 69 |
| 40 | Cl | Cl | 3 | $4-CH_3$ | 1 | |
| 41 | H | Cl | 2 | $2-OCH_3$ | 1 | |
| 42 | Br | Br | 2 | 4-F | 1 | |
| 43 | Br | Br | 2 | 2-F, 4-Cl | 2 | |
| 44 | Br | Br | 2 | $4-CH_3$ | 1 | |
| 45 | Cl | Cl | 2 | 3-Br, 4-F | 2 | 125 - 126 |
| 46 | Cl | Cl | 2 | 4-(4-Cl-Phenyl) | 1 | 170 - 173 |
| 47 | Cl | Cl | 2 | 4-Cyclopentyl | 1 | 127 - 128 |
| 48 | Cl | Cl | 2 | $3,4-(CH_3)_2$ | 2 | 156 - 157 |
| 49 | Cl | Cl | 2 | $3,4-(OH)_2$ | 2 | 164 - 165 |
| 50 | Br | Br | 2 | $2,4-F_2$ | 2 | 144 - 145 |
| 51 | Br | Br | 2 | $3-CF_3$ | 1 | 111 - 112 |
| 52 | Br | Br | 2 | 3-Cl | 1 | 159 - 160 |
| 53 | Br | Br | 2 | 2-F, 6-Cl | 2 | 145 - 146 |
| 54 | Br | Br | 2 | 2-Cl, 4-F | 2 | 167 - 168 |
| 55 | Br | Br | 2 | $2,3-Cl_2$ | 2 | 176 - 177 |
| 56 | Br | Br | 2 | $4-OCH_3$ | 1 | 152 - 153 |

*(Fortsetzung)*

| Beispiel-Nr. | $X_1$ | $X_2$ | n | R | m | Schmelzpunkt [°C] |
|---|---|---|---|---|---|---|
| 57 | Br | Br | 2 | 4-$CF_3$ | 1 | 145 - 146 |
| 58 | Br | Br | 2 | 4-$NO_2$ | 1 | 189 - 190 |
| 59 | Br | Br | 2 | 4-OH | 1 | 134 - 135 |
| 60 | Cl | Cl | 3 | 4-F | 1 | 71 - 74 |
| 61 | Cl | Cl | 3 | 3-Cl | 1 | 97 |
| 62 | Cl | Cl | 2 | 2,4-$Cl_2$, 5-F | 3 | 118 - 120 |
| 63 | Br | Br | 2 | 2,4-$Cl_2$, 5-F | 3 | 150 - 153 |
| 64 | Cl | Cl | 2 | 4-$N(CH_3)_2$ | 1 | 190 - 191 |
| 65 | H | Br | 2 | 4-F | 1 | |
| 66 | H | Br | 3 | 3-Cl | 1 | |
| 67 | H | Br | 2 | 2,6-$Cl_2$ | 2 | |

*Anwendungsbeispiele*

In den nachfolgenden Beispielen werden die nachstehend angegebenen Verbindungen als Vergleichssubstanzen eingesetzt:

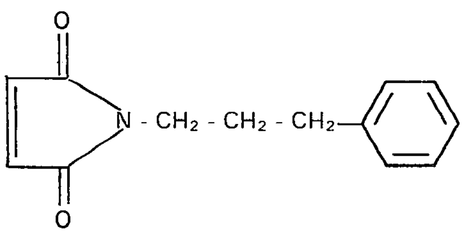
(A)

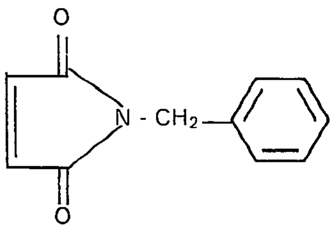
(B)

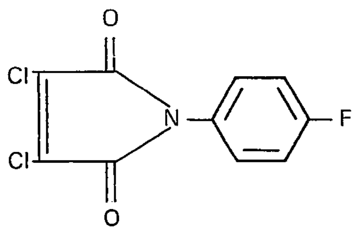
(C)

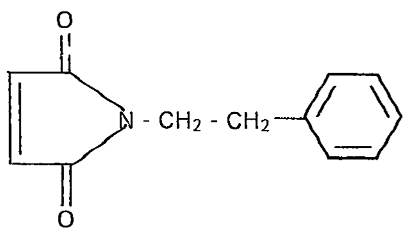
(D)

*Beispiel A*

Puccinia-Test (Weizen) / protektiv

Lösungsmittel:
100 Gewichtsteile Dimethylformamid.
Emulgator:
0,25 Gewichtsteile Alkylarylpolyglykolether.

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1%igen wässrigen Agarlösung inokuliert. Nach Antrocknen besprüht man die Pflanzen mit der Wirkstoffzubereitung taufeucht. Die Pflanzen verbleiben 24 Stunden bei 20°C und 100% rel. Luftfeuchtigkeit in einer Inkubationskabine.

Die Pflanzen werden in einem Gewächshaus bei einer Temperatur von ca. 20°C und einer relativen Luftfeuchtigkeit von ca. 80% aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäss folgender Herstellungsbeispiele: 6, 4, 8, 1.

*Beispiel B*

Leptosphaeria nodorum-Test (Weizen) / protektiv

Lösungsmittel:
100 Gewichtsteile Dimethylformamid.
Emulgator:
0,25 Gewichtsteile Alkylarylpolyglykolether.

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100% relativer Luftfeuchtigkeit in einer Inkubationskabine.

Die Pflanzen werden in einem Gewächshaus bei einer Temperatur von ca. 15°C und einer relativen Luftfeuchtigkeit von ca. 80% aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäss folgender Herstellungsbeispiele: 2, 11, 31, 8, 4, 1 und 30.

*Beispiel C*

Phytophthora-Test (Tomate) / protektiv

Lösungsmittel:
4,7 Gewichtsteile Aceton.
Emulgator:
0,3 Gewichtsteile Alkylarylpolyglykolether.

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wässrigen Sporensuspension von Phytophthora infestans inokuliert.

Die Pflanzen werden in einer Inkubationskabine mit 100% relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäss folgender Herstellungsbeispiele: 11, 18, 9, 16, 31, 27, 22, 30.

*Beispiel D*

Venturia-Test (Apfel) / protektiv

Lösungsmittel:
4,7 Gewichtsteile Aceton.
Emulgator:
0,3 Gewichtsteile Alkylarylpolyglykolether.

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wässrigen Konidiensuspension des Apfelschorferregers (Venturia inaequalis) inokuliert und verbleiben dann 1 Tag bei 20°C und 100% relativer Luftfeuchtigkeit in einer Inkubationskabine.

Die Pflanzen werden dann im Gewächshaus bei 20°C und einer relativen Luftfeuchtigkeit von ca. 70% aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäss folgender Herstellungsbeispiele: 33, 11, 6, 18, 16, 19, 31.

*Beispiel E*

Pyricularia-Test (Reis) / protektiv

Lösungsmittel:
12,5 Gewichtsteile Aceton.
Emulgator:
0,3 Gewichtsteile Alkylarylpolyglykolether.

Zur Herstellung einer zweckmässigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wässrigen Sporensuspension von Pyricularia oryzae inokuliert. Anschliessend werden die Pflanzen in einem Gewächshaus bei 100% rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäss folgender Herstellungsbeispiele: 19, 31.

*Beispiel F*

Pyricularia-Test (Reis) / systemisch

Lösungsmittel:
12,5 Gewichtsteile Aceton.
Emulgator:
0,3 Gewichtsteile Alkylarylpolyglykolether.

Zur Herstellung einer zweckmässigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wässrigen Sporensuspension von Pyricularia oryzae inokuliert. Danach verbleiben die Pflanzen in einem Gewächshaus bei einer Temperatur von 25°C und einer rel. Luftfeuchtigkeit von 100% bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäss folgender Herstellungsbeispiele: 18, 9, 16, 30.

*Beispiel G*

Pellicularia-Test (Reis)

Lösungsmittel:
12,5 Gewichtsteile Aceton.
Emulgator:
0,3 Gewichtsteile Alkylarylpolyglykolether.

Zur Herstellung einer zweckmässigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnass gespritzt. Die Pflanzen verbleiben bis zum Abtrocknen im Gewächshaus. Anschliessend werden die Pflanzen mit Pellicularia sasakii inokuliert und bei 25°C und 100% relativer Luftfeuchtigkeit aufgestellt.

5 bis 8 Tage nach der Inokulation erfolgt die Auswertung des Krankheitsbefalles.

Eine deutliche Überlegenheit in der Wirksamkeit gegenüber dem Stand der Technik zeigen bei diesem Test z.B. die Verbindungen gemäss folgender Herstellungsbeispiele: 11, 6, 32, 27.

**Patentansprüche**

1. Substituierte Maleinsäureimide der Formel (I)

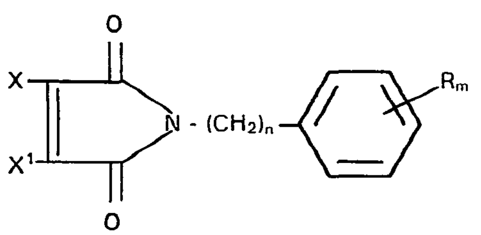

(I)

in welcher

X für Wasserstoff, Chlor oder Brom,

$X^1$ für Chlor oder Brom,

n für 2, 3 oder 4,

R für Halogen, Alkyl mit 1 bis 6 Kohlenstoffatomen, gegebenenfalls ein- bis fünffach, gleich oder verschieden durch Halogen und Alkyl substituiertes Aryl, Cycloalkyl oder Aryloxy, für Alkoxy, Alkoxycarbonyl, Alkylcarbonyl oder Alkylsulfonyl mit 1 bis 5 Kohlenstoffatomen je Alkylrest, für Carboxy, Aminocarbonyl, Aminosulfonyl, Nitro, Cyan, Hydroxy, Halogenalkyl mit 1 bis 7 Halogenatomen und 1 bis 4 Kohlenstoffatomen je Alkylrest, für Acyloxy oder Acylamino mit 1 bis 5 Kohlenstoffatomen je Acylrest, Amino, Mono- und Dialkylamino mit 1 bis 4 Kohlenstoffatomen je Alkylrest und

m für 1, 2, 3, 4 oder 5 stehen.

2. Substituierte Maleinsäureimide gemäss Anspruch 1, wobei in der Formel (I)

X für Wasserstoff, Chlor oder Brom,

$X^1$ für Chlor oder Brom,

n für 2, 3 oder 4,

R für Fluor, Chlor, Brom, Alkyl mit 1 bis 5 Kohlenstoffatomen, für Alkoxy mit 1 bis 4 Kohlenstoffatomen, für Aminosulfonyl, Alkylsulfonyl bzw. Alkylcarbonyl mit 1 bis 3 Kohlenstoffatomen im Alkylteil, für Aminocarbonyl, für Halogenalkyl mit 1 bis 3 Kohlenstoffatomen und 1 bis 5 Halogenatomen, Phenyl, Cyclopentyl oder Cyclohexyl, für Alkoxycarbonyl mit 1 bis 4 Kohlenstoffatomen im Alkoxyteil, für Hydroxy, für Acyloxy bzw. Acylamino mit 1 bis 3 Kohlenstoffatomen je Acylrest, für Amino, Mono- bzw. Dialkylamino mit 1 bis 3 Kohlenstoffatomen je Alkylrest, Nitro, Chlorphenyl und

m für 1, 2, 3, 4 oder 5 stehen.

3. Substituierte Maleinsäureimide gemäss Anspruch 1, wobei in der Formel (I)

X und $X^1$ für Chlor oder Brom,

n für 2 oder 3,

R für Fluor, Chlor, Brom, Methyl, Ethyl, n-Propyl, iso-Propyl, n-Butyl, sec.-Butyl, iso-Butyl und tert.-Butyl, für Methoxy, Ethoxy, n-Propoxy und iso-Propoxy, für Aminosulfonyl, für Methylsulfonyl, Ethylsulfonyl, n-Propylsulfonyl, iso-Propylsulfonyl, Methylcarbonyl, Ethylcarbonyl, n-Propylcarbonyl und iso-Propylcarbonyl, für Aminocarbonyl, Trifluormethyl, Phenyl, Cyclopentyl oder Cyclohexyl, für Methoxycarbonyl, Ethoxycarbonyl, n-Propoxycarbonyl und iso-Propoxycarbonyl, für Hydroxy, für Formyloxy, Acetoxy, Formylamino und Acetamido, für Amino, für Methylamino, Dimethylamino, Ethylamino, Diethylamino, Nitro und

m für 1, 2 oder 3 stehen.

4. Verfahren zur Herstellung von substituierten Maleinsäureimiden der Formel (I)

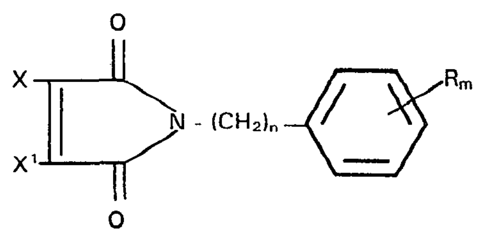

(I)

in welcher

X für Wasserstoff, Chlor oder Brom,

$X^1$ für Chlor oder Brom,

n für 2, 3 oder 4,

R für Halogen, Alkyl mit 1 bis 6 Kohlenstoffatomen, gegebenenfalls ein- bis fünffach, gleich oder verschieden durch Halogen und Alkyl substituiertes Aryl, Cycloalkyl oder Aryloxy, für Alkoxy, Alkoxycarbonyl, Alkylcarbonyl oder Alkylsulfonyl mit 1 bis 5 Kohlenstoffatomen je Alkylrest, für Carboxy, Aminocarbonyl, Aminosulfonyl, Nitro, Cyan, Hydroxy, Halogenalkyl mit 1 bis 7 Halogenatomen und 1 bis 4 Kohlenstoffatomen je Alkylrest, für Acyloxy oder Acylamino mit 1 bis 5 Kohlenstoffatomen je Acylrest, Amino, Mono- und Dialkylamino mit 1 bis 4 Kohlenstoffatomen je Alkylrest und

m für 1, 2, 3, 4 oder 5 steht, dadurch gekennzeichnet, dass man

a) ein Halogenmaleinsäureanhydrid der Formel (II)

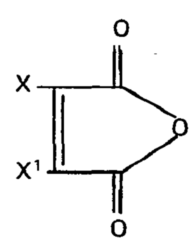

(II)

in welcher

X und $X^1$ die oben angegebene Bedeutung haben, mit primären Aminen der Formel (III)

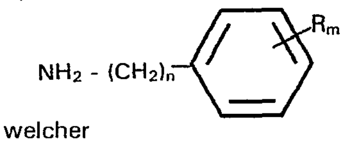
(III)

in welcher

R, m und n die oben angegebene Bedeutung haben, in einem Verdünnungsmittel umsetzt, oder

b) einen Halogenmaleinsäuredialkylester der Formel (IV)

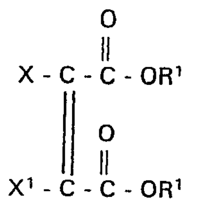
(IV),

in welcher

X und $X^1$ die oben angegebene Bedeutung haben, und

$R^1$ für Alkyl steht,

mit primären Aminen der Formel (III), in welcher

R, m und n die oben angegebene Bedeutung haben, gegebenenfalls in einem Lösungs- oder Verdünnungsmittel umsetzt, oder

c) Halogenmaleinsäuremonoamide der Formel (V)

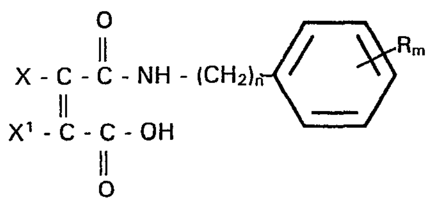
(V)

in welcher

X, $X^1$, R, m und n die oben angegebene Bedeutung haben,

in Gegenwart eines Lösungsmittels und gegebenenfalls in Gegenwart eines anhydridisierenden Mittels zu den Verbindungen der Formel (I) cyclisiert.

5. Schädlingsbekämpfungsmittel, gekennzeichnet durch einen Gehalt an mindestens einem substituierten Maleinsäureimid der Formel (I).

6. Verwendung von substituierten Maleinsäureimiden der Formel (I) zur Bekämpfung von Schädlingen.

7. Verfahren zur Bekämpfung von Schädlingen, dadurch gekennzeichnet, dass man substituierte Maleinsäureimide der Formel (I) auf Schädlinge und/oder ihren Lebensraum einwirken lässt.

8. Verfahren zur Herstellung von Schädlingsbekämpfungsmitteln, dadurch gekennzeichnet, dass man substituierte Maleinsäureimide der Formel (I) mit Streckmitteln und/oder oberflächenaktiven Mitteln vermischt.

**Claims**

1. Substituted maleic imides of the formula (I)

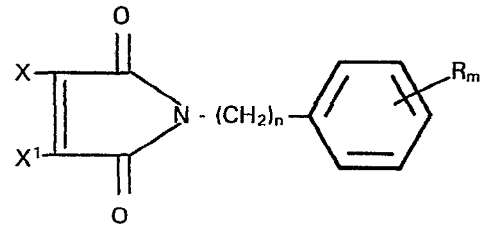
(I)

in which

X represents hydrogen, chlorine or bromine,

$X^1$ represents chlorine or bromine,

n represents 2, 3 or 4,

R represents halogen, alkyl with 1 to 6 carbon atoms, aryl, cycloalkyl or aryloxy, optionally mono- to penta-substituted by identical or different substituents from the group comprising halogen and alkyl; alkoxy, alkoxycarbonyl, alkylcarbonyl or alkylsulphonyl with 1 to 5 carbon atoms per alkyl radical, carboxyl, aminocarbonyl, aminosulphonyl, nitro, cyano, hydroxyl, halogenoalkyl with 1 to 7 halogen atoms and 1 to 4 carbon atoms per alkyl radical, acyloxy or acylamino with 1 to 5 carbon atoms per acyl radical, amino or mono- and dialkylamino with 1 to 4 carbon atoms per alkyl radical and

m represents 1, 2, 3, 4 or 5.

2. Substituted maleic imides according to claim 1, wherein in formula (I)

X represents hydrogen, chlorine or bromine,

$X^1$ represents chlorine or bromine,

n represents 2, 3 or 4,

R represents fluorine, chlorine, bromine, alkyl with 1 to 5 carbon atoms, alkoxy with 1 to 4 carbon atoms, aminosulphonyl, alkylsulphonyl, or alkylcarbonyl with 1 to 3 carbon atoms in the alkyl part, aminocarbonyl, halogenoalkyl with 1 to 3 carbon atoms and 1 to 5 halogen atoms, phenyl, cyclopentyl or cyclohexyl, alkoxycarbonyl with 1 to 4 carbon atoms in the alkoxy part, hydroxyl, acyloxy or acylamino with 1 to 3 carbon atoms per acyl radical, amino, mono- or dialkylamino with 1 to 3 carbon atoms per alkyl radical, nitro or chlorophenyl and

m represents 1, 2, 3, 4 or 5.

3. Substituted maleic imides according to claim 1, wherein in formula (I)

X and $X^1$ represents chlorine or bromine,

n represents 2 or 3,

R represents fluorine, chlorine, bromine, methyl, ethyl, n-propyl, iso-propyl, n-butyl, sec.-butyl, iso-butyl and tert.-butyl, methoxy, ethoxy, n-propoxy and iso-propoxy, aminosulphonyl, methylsulphonyl, ethylsulphonyl, n-propylsulphonyl, iso-propylsulphonyl, methylcarbonyl, ethylcarbonyl, n-propylcarbonyl and iso-propylcarbonyl, aminocarbonyl, trifluoromethyl, phenyl, cyclopentyl or cyclohexyl, methoxycarbonyl, ethoxycarbonyl, n-propoxycarbonyl and iso-propoxycarbonyl, hydroxyl, formyloxy, acetoxy, formylamino and acetamido, amino, methylamino, dimethylamino, ethylamino, diethylamino or nitro and

m represents 1, 2 or 3.

4. Process for the preparation of substituted maleic imides of the formula (I)

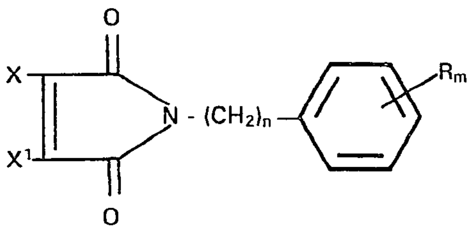

(I)

in which

X represents hydrogen, chlorine or bromine,

$X^1$ represents chlorine or bromine,

n represents 2, 3 or 4,

R represents halogen, alkyl with 1 to 6 carbon atoms, aryl, cycloalkyl or aryloxy, optionally mono- to penta-substituted by identical or different substituents from the group comprising halogen and alkyl; alkoxy, alkoxycarbonyl, alkylcarbonyl or alkylsulphonyl with 1 to 5 carbon atoms per alkyl radical, carboxyl, aminocarbonyl, aminosulphonyl, nitro, cyano, hydroxyl, halogenoalkyl with 1 to 7 halogen atoms and 1 to 4 carbon atoms per alkyl radical, acyloxy or acylamino with 1 to 5 carbon atoms per acyl radical, amino or mono- and dialkylamino with 1 to 4 carbon atoms per alkyl radical and

m represents 1, 2, 3, 4 or 5,

characterised in that

a) a halogenomaleic anhydride of the formula (II)

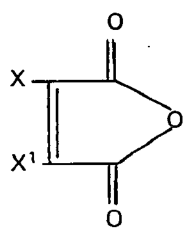

(II)

in which

X and $X^1$ have the abovementioned meaning, is reacted with primary amines of the formula (III)

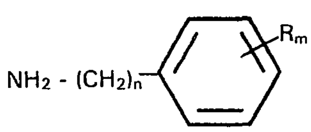

(III)

in which

R, m and n have the abovementioned meaning, in a diluent, or

b) a dialkyl halogenomaleate of the formula (IV)

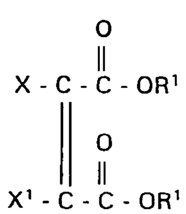

(IV)

in which

X and $X^1$ have the abovementioned meaning and

$R^1$ represents alkyl,

is reacted with primary amines of the formula (III) in which

R, m and n have the abovementioned meaning, if appropriate in a solvent or diluent, or

c) halogenomaleic acid monoamides of the formula (V)

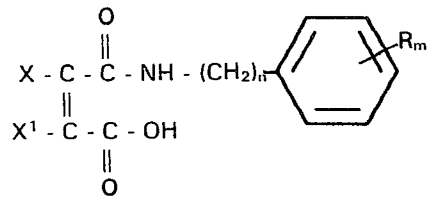

(V)

in which

X, $X^1$, R, m and n have the abovementioned meaning, are cyclised in the presence of a solvent and if appropriate in the presence of an anhydrising agent, to give the compounds of the formula (I).

5. Agents for combating pests, characterised in that they contain at least one substituted maleic imide of the formula (I).

6. Use of substituted maleic imides of the formula (I) for combating pests.

7. Method of combating pests, characterised in that substituted maleic imides of the formula (I) are allowed to act on pests and/or their environment.

8. Process for the preparation of agents for combating pests, characterised in that substituted maleic imides of the formula (I) are mixed with extenders and/or surface-active agents.

## Revendications

1. Imides d'acide maléique substitués de formule (I):

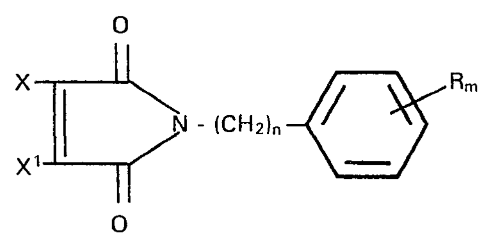

(I)

dans laquelle

X représente un atome d'hydrogène, un atome de chlore ou un atome de brome,

$X^1$ représente un atome de chlore ou un atome de brome,

n représente 2, 3 ou 4,

R représente un atome d'halogène, un groupe alkyle contenant 1 à 6 atomes de carbone, un groupe aryloxy, un groupe cycloalkyle ou un groupe aryle éventuellement substitué une à cinq fois de manière identique ou différente par un atome d'halogène et par un groupe alkyle; un groupe alcoxy, un groupe alcoxy-carbonyle, un groupe alkyl-carbonyle ou un groupe alkyl-sulfonyle contenant 1 à 5 atomes de carbone dans chaque groupe alkyle, un groupe carboxy, un groupe amino-carbonyle, un groupe amino-sulfonyle, un groupe nitro, un groupe cyano, un groupe hydroxy, un groupe halogénalkyle contenant 1 à 7 atomes d'halogènes et 1 à 4 atomes de carbone

dans chaque groupe alkyle, un groupe acyloxy ou un groupe acylamino contenant 1 à 5 atomes de carbone dans chaque groupe acyle, un groupe amino, un groupe mono- et dialkylamino contenant 1 à 4 atomes de carbone dans chaque groupe alkyle, et

m représente 1, 2, 3, 4 ou 5.

2. Imides d'acide maléique substitués selon la revendication 1, caractérisés en ce que, dans la formule (I),

X représente un atome d'hydrogène, un atome de chlore ou un atome de brome,

$X^1$ représente un atome de chlore ou un atome de brome,

n représente 2, 3 ou 4,

R représente un atome de fluor, un atome de chlore, un atome de brome, un groupe alkyle contenant 1 à 5 atomes de carbone, un groupe alcoxy contenant 1 à 4 atomes de carbone, un groupe amino-sulfonyle, un groupe alkyl-sulfonyle ou un groupe alkyl-carbonyle contenant 1 à 3 atomes de carbone dans la fraction alkyle, un groupe amino-carbonyle, un groupe halogénalkyle contenant 1 à 4 atomes de carbone et 1 à 5 atomes d'halogènes, un groupe phényle, un groupe cyclopentyle ou un groupe cyclohexyle, un groupe alcoxy-carbonyle contenant 1 à 4 atomes de carbone dans la fraction alcoxy, un groupe hydroxy, un groupe acyloxy ou un groupe acylamino contenant 1 à 3 atomes de carbone dans chaque groupe acyle, un groupe amino, un groupe mono- ou dialkylamino contenant 1 à 3 atomes de carbone dans chaque groupe alkyle, un groupe nitro ou un groupe chlorophényle, et

m représente 1, 2, 3, 4 ou 5.

3. Imides d'acide maléique substitués selon la revendication 1, caractérisés en ce que, dans la formule (I),

X et $X^1$ représentent chacun un atome de chlore ou un atome de brome,

n représente 2 ou 3,

R représente un atome de fluor, un atome de chlore, un atome de brome, un groupe méthyle, un groupe éthyle, un groupe n-propyle, un groupe isopropyle, un groupe n-butyle, un groupe sec-butyle, un groupe isobutyle et un groupe tert-butyle, un groupe méthoxy, un groupe éthoxy, un groupe n-propoxy et un groupe isopropoxy, un groupe amino-sulfonyle, un groupe méthyl-sulfonyle, un groupe éthyl-sulfonyle, un groupe n-propyl-sulfonyle, un groupe isopropylsulfonyle, un groupe méthyl-carbonyle, un groupe éthyl-carbonyle, un groupe n-propyl-carbonyle et un groupe isopropyl-carbonyle, un groupe amino-carbonyle, un groupe trifluorométhyle, un groupe phényle, un groupe cyclopentyle ou un groupe cyclohexyle, un groupe méthoxy-carbonyle, un groupe éthoxy-carbonyle, un groupe n-propoxy-carbonyle et un groupe isopropoxy-carbonyle, un groupe hydroxy, un groupe formyloxy, un groupe acétoxy, un groupe formylamino et un groupe acétamido, un groupe amino, un groupe méthylamino, un groupe diméthylamino, un groupe éthylamino, un groupe diéthylamino ou un groupe nitro, et

m représente 1, 2 ou 3.

4. Procédé de préparation d'imides d'acide maléique substitués de formule (I):

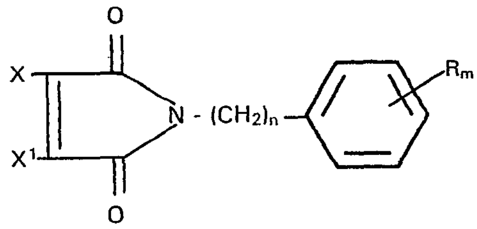

(I)

dans laquelle

X représente un atome d'hydrogène, un atome de chlore ou un atome de brome,

$X^1$ représente un atome de chlore ou un atome de brome,

n représente 2, 3 ou 4,

R représente un atome d'halogène, un groupe alkyle contenant 1 à 6 atomes de carbone, un groupe aryloxy, un groupe cycloalkyle ou un groupe aryle éventuellement substitué 1 à 5 fois de manière identique ou différente par un atome d'halogène et par un groupe alkyle; un groupe alcoxy, un groupe alcoxy-carbonyle, un groupe alkyl-carbonyle pu un groupe alkyl-sulfonyle contenant 1 à 5 atomes de carbone dans chaque groupe alkyle, un groupe carboxy, un groupe amino-carbonyle, un groupe amino-sulfonyle, un groupe nitro, un groupe cyano, un groupe hydroxy, un groupe halogénalkyle contenant 1 à 7 atomes d'halogènes et 1 à 4 atomes de carbone dans chaque groupe alkyle, un groupe acyloxy ou un groupe acylamino contenant 1 à 5 atomes de carbone dans chaque groupe acyle, un groupe amino, un groupe mono- et dialkylamino contenant 1 à 4 atomes de carbone dans chaque groupe alkyle, et

m représente 1, 2, 3, 4 ou 5,

caractérisé en ce que:

a) on fait réagir un anhydride d'acide halogénomaléique de formule (II):

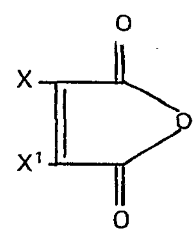

(II)

dans laquelle

X et $X^1$ ont les significations indiquées ci-dessus, avec des amines primaires de formule (III):

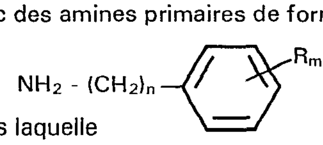

(III)

dans laquelle

R, m et n ont les significations indiquées ci-dessus, dans un diluant, ou

b) on fait réagir un ester dialkylique d'un acide halogénomaléique de formule (IV):

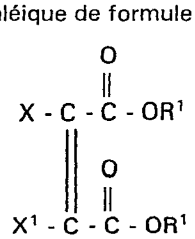

(IV),

dans laquelle

X et $X^1$ ont les significations indiquées ci-dessus, et

$R^1$ représente un groupe alkyle,

avec des amines primaires de formule (III) dans laquelle

R, m et n ont les significations indiquées ci-dessus, éventuellement dans un solvant ou dans un diluant, ou

c) on cyclise des mono-amides d'acides halogéno-maléiques de formule (V):

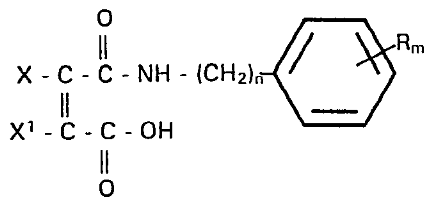

(V)

dans laquelle

X, $X^1$, R, m et n ont les significations indiquées ci-dessus,

en présence d'un solvant et éventuellement en présence d'un agent formateur d'anhydride pour obtenir les composés de formule (I).

5. Parasiticides, caractérisés en ce qu'ils contiennent au moins un imide d'acide maléique substitué de formule (I).

6. Utilisation d'imides d'acide maléique substitués de formule (I) pour combattre les parasites.

7. Procédé en vue de combattre les parasites, caractérisé en ce qu'on fait agir des imides d'acide maléique substitués de formule (I) sur les parasites et/ou leur biotope.

8. Procédé de préparation de parasiticides, caractérisé en ce qu'on mélange des imides d'acide maléique substitués de formule (I) avec des diluants et/ou des agents tensio-actifs.